# EUROPEAN PATENT APPLICATION

(11) **EP 3 750 493 A1**
(43) Date of publication of application: **16.12.2020**
(21) Application number: 19750405.3
(22) Date of filing: 06.02.2019
(51) Int. Cl.: A61B 17/24, A61B 5/107, A61F 2/20

(54) **THYROID CARTILAGE-SHAPE MEASUREMENT JIG**

(30) Priority: 06.02.2018 JP 2018019697
(71) Applicant: Nobelpharma Co., Ltd., Chuo-ku Tokyo 103-0024 (JP)
(72) Inventor: ABURADA, Takako, Tokyo 103-0024 (JP)
(74) Representative: Schön, Christoph
(86) International application number: PCT/JP2019/004307
(87) International publication number: WO 2019/156132

(57) **Abstract**

The present invention provides a jig for measuring the shape of the thyroid cartilage capable of readily measuring the shape of the front surface of the thyroid cartilage. The jig 20 for measuring the shape of the thyroid cartilage of the present invention includes: a display section 22 having a plurality of relatively slidable movable members 21 and configured to display a shape of thyroid cartilage 11 by contacting distal end surfaces 21t of the movable members 21 with a front surface 11a of the thyroid cartilage 11; and a maintenance section 23 configured to align and maintain the movable members 21 in one direction and capable of fixing the slid movable members 21. Two of such display sections 22 may be provided in the one direction with a space.

## Description

### Technical Field

The present invention relates to a jig for measuring the shape of the thyroid cartilage.

### Background Art

To improve spasmodic dysphonia including a state of the vocal cords failed to vibrate due to excessive glottic closure, a dysphonia treatment tool described in, for example, PTL 1 below is proposed. The dysphonia treatment tool disclosed in PTL 1 includes two titanium clamping sections clamping cut ends on both sides of incised thyroid cartilage and a titanium bridging section bridging the two clamping sections to maintain an incision space of the incised thyroid cartilage. Each of the clamping sections includes a front piece disposed on a front surface side of the incised thyroid cartilage and a rear piece disposed on a rear surface side of the thyroid cartilage.

Then, the thyroid cartilage is incised in the midline, the cut ends of the thyroid cartilage are separated by forceps and the like, the clamping sections of the dysphonia treatment tool are fit to the separated cut ends facing each other, and the forceps are removed. Then, the separated cut ends elastically return in a closing direction, and thus the dysphonia treatment tool is securely fixed between the cut ends.

Further, a suture thread is passed through holes formed in the front pieces for the purpose of gripping the dysphonia treatment tool or the like to suture the dysphonia treatment tool to the thyroid cartilage. This allows more reliable prevention of displacement of the placed dysphonia treatment tool at the cut ends. As described above, it is possible to reliably fix the dysphonia treatment tool between the separated thyroid cartilage.

A position in the thyroid cartilage to place the treatment tool is often an upper end or a lower end of the midline incision in the thyroid cartilage. The shape of the thyroid cartilage differs from patient to patient. In particular, the projecting angle or the inclination angle of the thyroid cartilage differs by sex or race. Accordingly, the treatment tool is expected to have the front pieces of the clamping sections to be bent along the shape of the front surface of the thyroid cartilage and the holes to be located near the thyroid cartilage as close as possible to facilitate fixation by suture. It is thus difficult to uniformly set, in advance, the bending condition of the treatment tool placed on the thyroid cartilage and it is preferred to set the bending condition in accordance with the shape of the cartilage for each patient.

### Citation List

### Patent Literature

PTL 1: JP 2005-330 A

### Summary of Invention

### Technical Problem

However, it is difficult to recognize the shape of the thyroid cartilage only by incising the throat and viewing the thyroid cartilage from the front. Although the shape of the thyroid cartilage may be considered to be specified by placing a molding gel, such as plastics, on the thyroid cartilage, it also has to be considered that mold making takes time and is not preferred and the gel component exerts an influence on the site. Although it is also possible to make a mold by placing a material with a readily changing shape, such as a copper wire, on the front surface of the thyroid cartilage, mold making by placement on the thyroid cartilage has a concern for exerting an influence on the thyroid cartilage and the peripheral tissues. Metal materials, such as a copper wire, somewhat elastically returns and thus cause difficulty in precise mold making.

It is thus an object of the present invention to provide a jig for measuring the shape of the thyroid cartilage capable of conveniently measuring the shape of the front surface of the thyroid cartilage.

### Solution to Problem

A jig for measuring a shape of thyroid cartilage of the present invention includes:
a display section having a plurality of relatively slidable movable members and configured to display a shape of thyroid cartilage by contacting distal end surfaces of the plurality of movable members with a front surface of the thyroid cartilage; and
a maintenance section configured to align and maintain the movable members in one direction and capable of fixing the slid movable members.

This configuration allows easy measurement of the shape of the front surface of the thyroid cartilage.

In the jig for measuring a shape of thyroid cartilage of the present invention, two of the display section may be provided in the one direction with a space.

This configuration allows recognition of both shapes of the cut ends of the incised thyroid cartilage at a time.

The jig for measuring a shape of thyroid cartilage of the present invention may further include a spacer configured to adjust a space between the two display sections.

This configuration allows arbitrary setting of the space between the display sections in accordance with the length of the bridging section of the treatment tool placed in the thyroid cartilage after measuring the shape of the thyroid cartilage to facilitate tracing the shape to the bending condition of the front pieces of the treatment tool.

In the jig for measuring a shape of thyroid cartilage of the present invention, the movable members may be detachably provided to the maintenance section.

This configuration facilitates washing and sterilization after using the jig for measuring the shape of the thyroid cartilage.

In the jig for measuring a shape of thyroid cartilage of the present invention, the movable members aligned in the maintenance section may have a thickness dimension of 0.5 mm or more and 5 mm or less.

This configuration facilitates precise recognition of the shape of the thyroid cartilage at the placement site.

In the jig for measuring a shape of thyroid cartilage of the present invention, the maintenance section may have, formed therein, an insertion hole formed with a groove portion to allow the movable members to slide or insertion holes to insert the movable members one by one.

This configuration facilitates handling of the movable members or allows, when one of the movable members is moved, following movement of the other movable members.

In the jig for measuring a shape of thyroid cartilage of the present invention, each movable member may have, formed therein, a long hole extending in a direction of relative movement of the movable members to each other, and
a ring member may be inserted into the long holes of the plurality of movable members configuring the display section.

This configuration allows collection of the movable members for each display section to facilitate handling of the movable members.

In the jig for measuring a shape of thyroid cartilage of the present invention,
the spacer may have a hole formed therein,
the spacer may be rotatably maintained by the ring member while the ring member is inserted into the hole of the spacer, and
the spacer may have a notch formed therein capable of engaging with an area different from an area of the ring member being inserted into the hole.

This configuration allows collection of the movable members for each display section with the ring member and linking of the spacer to the ring member to facilitate handling of the movable members and the spacer.

In the jig for measuring a shape of thyroid cartilage of the present invention, a side surface or a base end surface of any of the plurality of movable members may have a color different from the other movable members.

This configuration facilitates visual recognition of the movable members of the jig for measuring the shape of the thyroid cartilage to facilitate handling of the jig for measuring the shape of the thyroid cartilage.

In the jig for measuring a shape of thyroid cartilage of the present invention, each of the movable members may have a side surface with a scale marked with fixed intervals and linearly extending in a horizontal direction while base end surfaces or the distal end surfaces of the movable members.

This configuration facilitates calculation of the amount of relative movement of the movable members to each other with the scale on the side surface of the display section when the shape of the thyroid cartilage is traced.

### Advantageous Effects of Invention

The present invention exhibits an effect of allowing convenient measurement of the shape of the front surface of the thyroid cartilage.

### Brief Description of the Drawings

Fig. 1 is a diagram illustrating a state of using a jig for measuring the shape of the thyroid cartilage in an embodiment of the present invention.
Fig. 2 is a perspective view illustrating a dysphonia treatment tool reflecting a shape of the thyroid cartilage measured using the jig for measuring the shape of the thyroid cartilage of the present invention.
Fig. 3 is a diagram illustrating a state of placing the dysphonia treatment tool, in the thyroid cartilage, deformed using the jig for measuring the shape of the thyroid cartilage of the present invention.
Fig. 4A is a side view illustrating a movable member of the jig for measuring the shape of the thyroid cartilage of the present invention and Fig. 4B is a front view illustrating the movable member.
Fig. 5A is a side view illustrating the jig for measuring the shape of a thyroid cartilage in an embodiment of the present invention in a state of tracing the thyroid cartilage and disposing a spacer. Fig. 5B is a front view illustrating a jig for measuring the shape of the thyroid cartilage in an embodiment of the present invention in a state of tracing the thyroid cartilage and disposing a spacer.
Fig. 6 is a plan view illustrating a jig for measuring the shape of the thyroid cartilage in an embodiment of the present invention.
Fig. 7 is a side view illustrating a jig for measuring the shape of the thyroid cartilage in an embodiment of the present invention.
Fig. 8 is a side view illustrating a modification of a jig for measuring the shape of the thyroid cartilage in an embodiment of the present invention.
Fig. 9 is a side view illustrating a modification of a jig for measuring the shape of the thyroid cartilage in an embodiment of the present invention.
Fig. 10 is a plan view illustrating a modification of a maintenance section of a jig for measuring the shape of the thyroid cartilage in an embodiment of the present invention.
Fig. 11 is a side view illustrating a modification of a jig for measuring the shape of the thyroid cartilage in an embodiment of the present invention.
Fig. 12 is a side view illustrating a modification of a jig for measuring the shape of the thyroid cartilage in an embodiment of the present invention.
Fig. 13 is a plan view illustrating a modification of a maintenance section of a jig for measuring the shape of the thyroid cartilage in an embodiment of the present invention.
Fig. 14 is a side view illustrating a modification of a jig for measuring the shape of the thyroid cartilage in an embodiment of the present invention.
Fig. 15 is a front view illustrating a modification of a jig for measuring the shape of the thyroid cartilage in an embodiment of the present invention.
Figs. 16A-C are plan views illustrating a modification of a jig for measuring the shape of the thyroid cartilage in an embodiment of the present invention.

### Description of Embodiments

Embodiments of a jig for measuring the shape of the thyroid cartilage of the present invention are described with reference to the drawings together with a dysphonia treatment tool as an application object thereof.

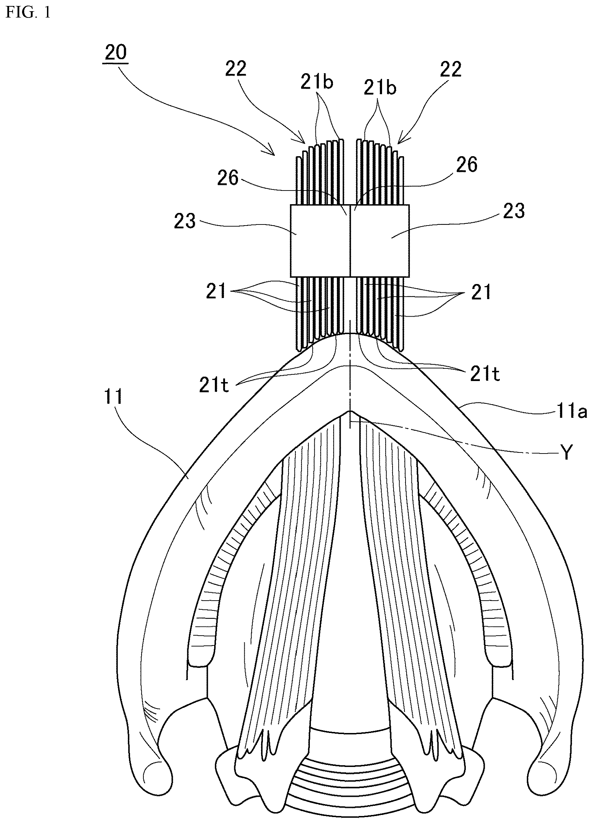

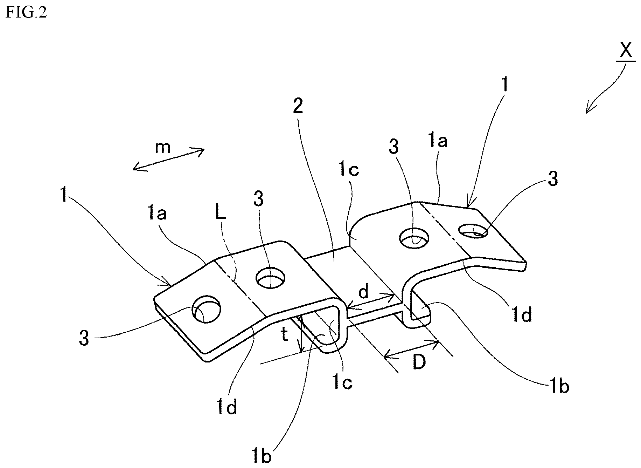

As illustrated in Fig. 1, a jig for measuring the shape of the thyroid cartilage (hereinafter, referred to as a "measurement jig") 20 in an embodiment of the present invention traces the shape of thyroid cartilage 11 to measure an angle to bend a front piece 1a of a dysphonia treatment tool X (hereinafter, referred to as a "treatment tool") illustrated in Fig. 2.

The treatment tool X is first described to be an application object of the jig 20 for measuring the shape.

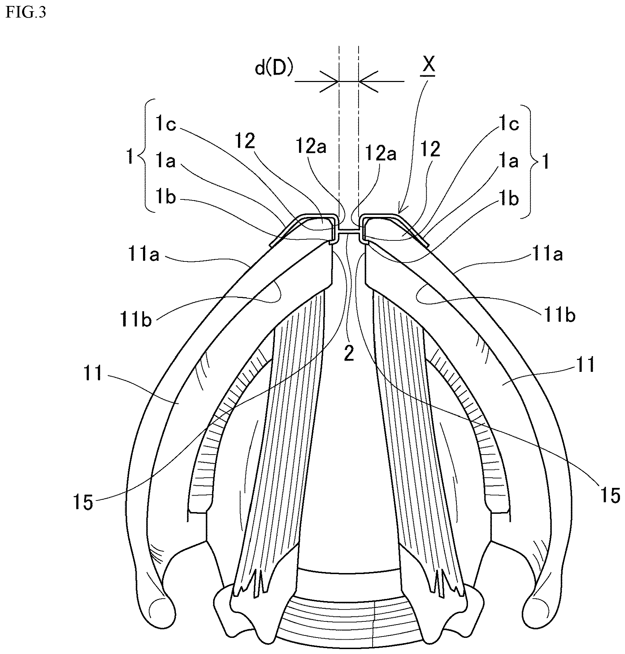

As illustrated in Fig. 2, the treatment tool X includes: a plurality (in the present embodiment, one pair) of clamping sections 1, 1 each having the front piece 1a and a rear piece 1b and being configured to be fit to respective cut ends 12, 12, facing each other, of the incised thyroid cartilage 11 illustrated in Fig. 3; and a bridging section 2 linking the clamping sections 1, 1.

The front piece 1a is formed in a substantially strip-like plate shape in a plan view. A base end side of the front piece 1a in a longitudinal direction is bent and forms an end surface portion 1c to be abut on a cut end surface 12a of the thyroid cartilage 11 illustrated in Fig. 3. A distal end side relative to the end surface portion 1c of the front piece 1a forms a surface placement portion 1d to be placed on a front surface 11a of the thyroid cartilage 11.

In the front piece 1a, a plurality (in the present embodiment, two) of holes 3 are formed at an interval in the longitudinal direction. The hole 3 on the base end side of the front piece 1a is formed at a position of approximately 2 mm from a base end edge in a plan view of the surface placement portion 1d, and the hole 3 on the distal end side is formed with a space of approximately 2 mm from the hole 3 on the base end side. The holes 3, 3 allow insertion of a suture thread (not shown, hereinafter the same) therein and are formed in the size from 1.0 mm to 2.0 mm to allow insertion of a suture needle therein.

As illustrated in Fig. 2 and Fig. 3, the rear piece 1b is bent from the end surface portion 1c of the front piece 1a towards a rear surface 11b side of the thyroid cartilage 11 illustrated in Fig. 3.

The front piece 1a and the rear piece 1b are integrally formed to constitute the clamping section 1, and the entire clamping section 1 is formed in a substantially J shape (inverted J shape).

In the clamping sections 1, 1, the front piece 1a may have a length that is necessary and sufficient for clamping the thyroid cartilage 11 and can extend along the form of the thyroid cartilage 11. Specifically, the length is preferably set approximately from 8 mm to 12 mm. The rear piece 1b preferably has a length to allow contact from an end edge of the cut end 12 of the thyroid cartilage 11 to an end edge of a soft tissue 15 under the thyroid cartilage 11. Specifically, the rear piece 1b preferably has a length approximately from 1.5 mm to 3.5 mm.

As illustrated in Fig. 3, the pair of clamping sections 1 is provided in bilateral symmetry each having the front piece 1a disposed on the front surface 11a side of the thyroid cartilage 11 and the cut end surface 12a and the rear piece 1b disposed on the rear surface 11b side of the thyroid cartilage 11 to allow fitting to the respective cut ends 12, 12 of the thyroid cartilage 11 facing each other. The pair of clamping sections 1, 1 are linked by the bridging section 2.

As illustrated in Fig. 2, the bridging section 2 is a section linking the clamping sections 1 and links the clamping sections 1 in an intermediate section in an extending direction of the end surface portion 1c. The bridging section 2 has a length d, that is, a space D between both clamping sections 1, 1 equivalent to a distance between the cut ends 12, 12 of the separated thyroid cartilage 11 illustrated in Fig. 3 and, while the length d differs depending on symptoms, body types, and vocal states of patients with dysphonia, the length d is generally set in the range from 2 to 6 mm.

In a preferred mode, the clamping sections 1 and the bridging section 2 of the treatment tool X are both composed of titanium.

A titanium metal used for the treatment tool X is not limited to titanium as a pure metal and includes titanium alloys used for artificial bones, artificial joints, and dental implants as biocompatible metal materials. Specifically, it is possible to use Ti-6Al-4V or the like that does not contain Ni, which is indicated as a cause of cancer and allergies, and is known as a titanium alloy excellent in biocompatibility. To prevent wear and elution, the titanium or the titanium alloy may be surface modified by ion implantation of N or C.

The front surface placement portion 1d and the rear piece 1b preferably have a space (width dimension of the end surface portion 1c) t between them slightly greater than a thickness dimension of the thyroid cartilage 11 illustrated in Fig. 3, and specifically the space t is preferably from 2 to 4 mm approximately. If the space t is less than the thickness dimension of the thyroid cartilage 11 illustrated in Fig. 3, the thyroid cartilage 11 is squeezed and the clamping sections 1 continually presses the thyroid cartilage 11 for a long period of time, causing a possibility of wear and damage to the thyroid cartilage 11. In contrast, if the space t between the surface placement portion 1d and the rear piece 1b is excessively large in comparison with the thickness dimension of the thyroid cartilage 11, it is substantially difficult to clamp the thyroid cartilage 11 and the clamping sections 1 are relatively liable to position shifting (sliding) with respect to the thyroid cartilage 11.

The configuration of the measurement jig 20 is then described.

As illustrated in Fig. 1, the measurement jig 20 includes: a display section 22 having a plurality of movable members 21 aligned in one direction with overlapped flat surfaces and configured to display a shape of thyroid cartilage by contacting the movable members 21 with a front surface 11a of the thyroid cartilage 11; and a maintenance section 23 configured to maintain the movable members 21 and capable of fixing the movable members 21.

In the present embodiment, two of the display sections 22 are provided in the direction of aligning the movable members 21 with a space.

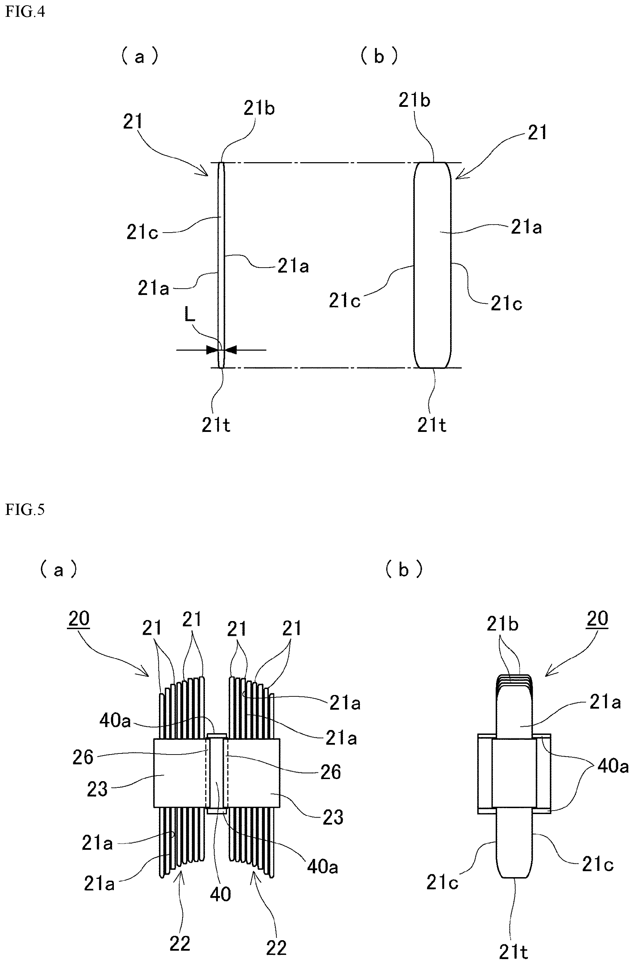

As illustrated in Figs. 4A and 4B, each movable member 21 is formed in a flat and substantially rectangular plate shape. The movable member 21 has flat surfaces 21a, 21a, side surfaces 21c, 21c, a distal end surface 21t, and a base end surface 21b.

As illustrated in Fig. 5A, each display section 22 is formed by overlapping the plurality of movable members 21 to face the flat surfaces 21a of the movable members 21.

Each display section 22 includes, although depending on the thickness dimension of each movable member 21, two or more of the movable members 21, for example, to allow placement on one of two regions on the left and right divided by an imaginary cut line Y on the front surface 11a of the thyroid cartilage 11 illustrated in Fig. 1 intended to be traced. Specifically, the display section 22 may include the number of the movable members 21 to have the entire length in the direction of aligning the movable members 21 of 3 mm or more and preferably 6 mm or more. For example, when each movable member 21 has a thickness of 1 mm, the display section 22 may include three or more, preferably from 6 to 10, of the movable members 21. In the present embodiment, each display section 22 is configured by including eight movable members 21 with a thickness of 1 mm.

Each movable member 21 has a thickness dimension L that is not particularly limited while a smaller thickness dimension L allows expression of a finer change in shape. The thickness dimension L is preferably 0.5 mm or more and 2 mm or less and more preferably set at 0.5 mm or 1 mm. Particularly, the movable member 21 with a thickness dimension L set at 0.5 mm or 1 mm facilitates calculation of a distance between a specified site (e.g., imaginary line Y, etc.) and the position of a change in shape.

Each flat surface 21a of the movable member 21 may have dimensions formed in size facilitating gripping of the movable member 21 and insertion into the incised opening from the perspective of the operability for placing the movable member 21 on the thyroid cartilage 11 and the size, the depth, and the like of the opening in the incised skin. The flat surface 21a of the movable member 21 preferably has a dimension in the transverse direction (i.e., lateral direction) of 2 mm or more and 10 mm or less and a dimension in the lengthwise direction (i.e., longitudinal direction) of 30 mm or more and 100 mm or less. The dimension in the transverse direction ranges from 2 mm or more to 10 mm or less in order to keep stability when the distal end surface 21t of the movable member 21 is placed on the front surface 11a of the thyroid cartilage 11 and to facilitate conformity to the unevenness on the front surface 11a of the thyroid cartilage 11. The dimension in the lengthwise direction ranges from 30 mm or more to 100 mm in order to secure the length reaching the thyroid cartilage 11 from the opening in the incised skin and facilitate handling by an operator, such as a doctor.

In the example illustrated in Fig. 4, the distal end surface 21t of the movable member 21 is formed in a smooth arc shape in a side view, in other words, a shape with rounded corners. The distal end surface 21t of the movable member 21 thus formed allows prevention of a wound and the like in the thyroid cartilage 11 by contacting with the front surface 11a of the thyroid cartilage 11. As a material for the movable member 21, it is possible to use a resin or a metal that is sterilizable and has rigidity less likely to be bent, modified, or damaged and to specifically use, for example, the same materials for SUS or the treatment tool X.

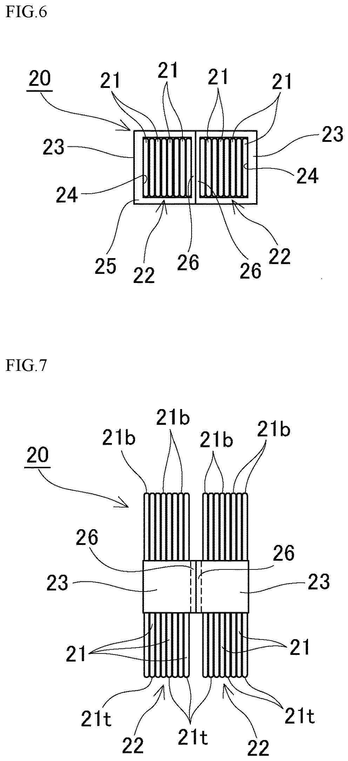

As illustrated in Fig. 6, the maintenance section 23 is a member in a frame shape having an insertion hole 24 in the size allowing maintenance of the plurality of movable members 21 configuring one of the display sections 22 to be relatively slidable on each other and not to allow unintentional movement. Two of such insertion holes 24 are formed in the direction of aligning the movable members 21 with a space.

Specifically, the maintenance sections 23 have: an outer wall portion 25 surrounding and maintaining the periphery of the display sections 22, that is, the periphery of the movable members 21 with the flat surfaces 21a overlapped with each other; and inner wall portions 26, 26 partitioning the two display sections 22, 22.

Each insertion hole 24 formed by the outer wall portion 25 and the inner wall portion 26 is formed in substantially the same shape and the same dimensions in a plan view of one of the display sections 22 to allow tight maintenance of the stacked movable members 21 to be slidable and not to unintentionally move.

As illustrated in Fig. 5A, the inner wall portions 26, 26 are separable to allow a spacer 40 to be disposed between them to adjust a space between the inner wall portions 26, 26. In the example illustrated in Figs. 5A and 5B, the inner wall portions 26, 26 include magnets at least in part to be detachable to each other. The inner wall portions 26, 26 may have a thickness set at a dimension of not more than a minimum width (approximately 1 mm) of a width dimension d of the bridging section 2 of the treatment tool X illustrated in Fig. 2.

The spacer 40 is a plate member configured to adjust the space between the maintenance sections 23, 23. The thickness of the spacer 40 is formed at, but not particularly limited to, 0.5 mm or 1 mm to allow adjustment of the space between the maintenance sections 23, 23 by 0.5 mm or 1 mm each. The spacer 40 may have an engagement portion 40a to be engaged with the end surfaces of the maintenance sections 23 in at least either one of the ends (in the present embodiment, both ends) in the longitudinal direction to allow attachment of the maintenance sections 23, 23 at the approximately same position with respect to the longitudinal direction of the spacer 40.

As illustrated in Fig. 5B, the spacer 40 has a dimension in the lateral direction (i.e., dimension in the direction corresponding to the transverse direction of the movable members 21) formed, but not particularly limited to, greater than the dimension in the corresponding transverse direction of the maintenance sections 23 to facilitate attachment of the movable members 21.

In the example illustrated in Figs. 5A and 5B, the spacer 40 is formed containing metal to allow attachment to magnet portions in the inner wall portions 26.

A description is then given to a method of using the measurement jig 20.

At first, as illustrated in Fig. 7, the maintenance sections 23, 23 of the measurement jig 20 are aligned and the inner wall portions 26, 26 are tightly attached to each other by the magnetic force of the magnets to align the distal end surfaces 21t and the base end surfaces 21b of the movable members 21 linearly in a side view. After that, as illustrated in Fig. 1, the measurement jig 20 is arranged to locate the borderline between the inner wall portions 26, 26 on the imaginary line Y on the front surface 11a to cut the thyroid cartilage 11. Then, the distal end surfaces 21t of the movable members 21 in the display sections 22 are contacted with the front surface 11a of the thyroid cartilage 11 and the movable members 21 loose from the front surface 11a are slid to contact the distal end surfaces 21t of all the movable members 21 with the front surface 11a.

After tracing the shape of the front surface 11a of the thyroid cartilage 11, the measurement jig 20 is removed from the thyroid cartilage 11 not to move the movable members 21 in this state. The thyroid cartilage 11 is incised as illustrated in Fig. 3 and the dimension to separate the cut ends 12, 12 from each other is determined. The spacer 40 with a thickness smaller than the determined dimension by the thickness of the inner wall portions 26, 26 is disposed between the inner wall portions 26, 26.

As described above, the shape of the front surface 11a of the thyroid cartilage 11 and the dimension between the cut ends 12, 12 are represented by the display sections 22, 22 and the spacer 40 of the measurement jig 20 and thus the left and right front pieces 1a, 1a of the treatment tool X are deformed in accordance with the shape drawn by the distal end surfaces 21t or the base end surfaces 21b of the movable members 21. The treatment tool X thus deformed allows placement of the deformed treatment tool X, as illustrated in Fig. 3, between the cut ends 12, 12 with a predetermined space to be fit to the front surface 11a of the thyroid cartilage 11.

After using the measurement jig 20, the movable members 21 are pulled out of the maintenance sections 23 to be taken apart and each is subjected to washing and sterilization.

The measurement jig 20 of the present invention thus exhibits an effect of allowing easy measurement with necessary and sufficient precision of the shape of the front surface 11a at the cut ends 12 of the thyroid cartilage 11 where the treatment tool X is to be placed.

The measurement jig 20 allows easy disassembly and assembly and thus exhibits an effect of allowing the measurement jig 20 to be sanitarily and repeatedly used by appropriate washing and sterilization of the jig 20.

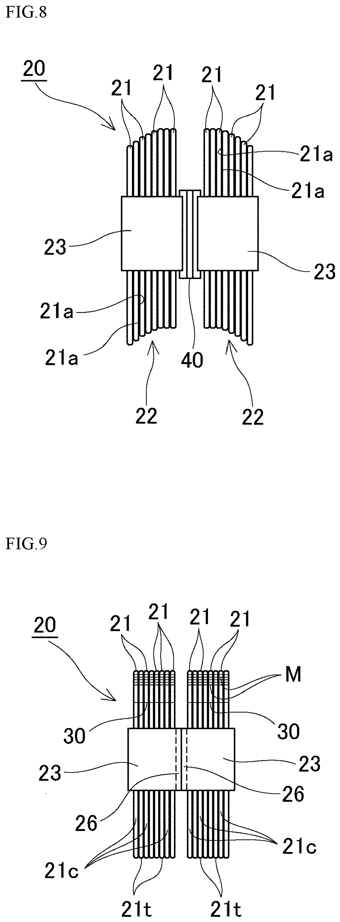

It should be noted that, although the above embodiment describes the example of using the one spacer 40 with the predetermined dimension set in advance in accordance with the dimension between the cut ends 12, 12, the spacer 40 may be formed, as illustrated in Fig. 8, by overlapping a plurality of plate members with a fixedly set thickness of, for example, 0.5 mm, 1 mm, or the like. The spacer 40 with such a configuration exhibits an effect of facilitating arbitrary setting the dimension between the inner wall portions 26, 26 in accordance with the separated dimension between the cut ends 12, 12 of the thyroid cartilage 11.

At least the base end surface 21b or the side surface 21c of any of the movable members 21 disposed near the inner wall portion 26 may have a color different from the other movable members 21. Such a configuration facilitates visual recognition of the center of the movable members 21, which are even smaller components in the relatively small measurement jig 20, and exhibits an effect of facilitating placement of the measurement jig 20 on the front surface 11a of the thyroid cartilage 11.

As illustrated in Fig. 9, the side surfaces 21c of the movable members 21 configuring the display section 22 may be provided with a linear indication 30 to form a straight line between the plurality of movable members 21 while the distal end surfaces 21t of the movable members 21 are aligned.

The side surfaces 21c with the linear indication 30 exhibits an effect of allowing expression of a result of tracing the shape of the front surface 11a of the thyroid cartilage 11 on the side surfaces 21c of the movable members 21.

As illustrated in Fig. 9, the side surfaces 21c forming the thickness of the movable members 21 may be provided with a scale M marked with fixed intervals and linearly extending in a horizontal direction between the movable members 21. Although the intervals of the scale M are not particularly limited, the mark M with intervals of, for example, 0.5 mm each allows easy recognition of the relative moved dimension among the movable members 21, 21.

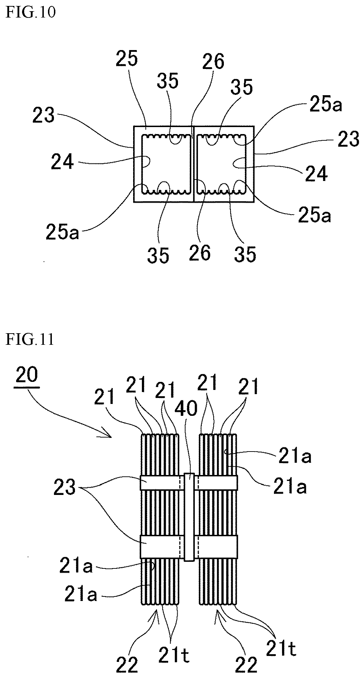

In the example illustrated in Fig. 6, the measurement jig 20 in the embodiment described above has the outer wall portion 25 forming the insertion hole 24 with an inner wall surface formed flat. However, as illustrated in Fig. 10, the insertion hole 24 may have an inner wall surface 25a formed with a groove portion 35 to slide the movable members 21 corresponding to the shape of the side surfaces 21c of the movable members 21. When such a groove portion 35 is formed, it is possible to prevent the movable members 21 from shaking in a direction different from the direction to slide during sliding of the movable members 21 and cause the movable members 21 to be slid in an axial direction of the insertion hole 24 of the maintenance section 23. This exhibits an effect of more precise measurement of the shape of the front surface 11a of the thyroid cartilage 11 by the measurement jig 20.

As illustrated in Fig. 11, a plurality of maintenance sections 23 may be placed with a space in the lengthwise direction of the movable members 21. In this case, the spacer 40 may be disposed either only in one of the maintenance sections 23 or across both maintenance sections 23. When the maintenance sections 23 are thus placed, it is possible to prevent a wobble in the movable members 21 during sliding and cause the distal end surfaces 21t of the movable members 21 to be stably contacted with the front surface 11a of the thyroid cartilage 11. It is also possible to more reliably maintain the movable members 21.

In this case, one of the maintenance sections 23 may be formed with a resin with a high coefficient of friction, such as silicon, or the like to form a fixation mechanism capable of squeezing the movable members 21 stepwise.

The maintenance sections 23 thus formed allow the maintenance section 23 to be a reliable fixation mechanism for the movable members 21 and allow prevention of unintentional movement of the movable members 21 after recognition of the shape of the front surface 11a of the thyroid cartilage 11. It should be noted that such a configuration may be applied to the case of providing only one maintenance section 23.

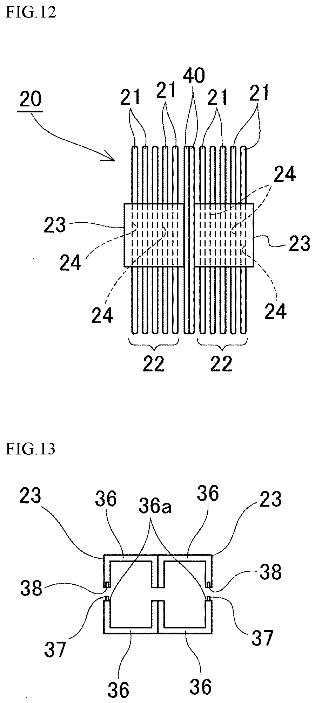

As another example illustrated in Fig. 12, the measurement jig 20 may have the maintenance section 23 with individual insertion holes 24 formed for each movable member 21. The dimension of each space between the insertion holes 24 may be set, but not particularly limited to, approximately from 0.5 mm to 1 mm. Each space between the insertion holes 24 thus set allows the measurement jig 20 to readily recognize the overall change in shape and the area where the bending condition of the front surface 11a of the thyroid cartilage 11 appears.

In addition, employment of the configuration in which the insertion hole 24 of the maintenance section 23 is formed for each movable member 21 to form a gap between the movable members 21 allows prevention of unintentional following movement of the adjacent movable members 21 while sliding the movable members 21. The movable members 21 apart from each other brings an effect of facilitating handling of the movable members 21 with a thickness set to be small.

It should be noted that, although the present modification exemplifies a configuration of forming the insertion hole 24 for each movable member 21 as the maintenance section 23, the maintenance section 23 may have, although not shown, a groove portion to slide the movable members 21 formed in the inner wall surface of the maintenance section 23 and a gap may be formed between the groove portions to form a gap between the movable members 21.

Although the above embodiment and the above modifications describe the examples of providing the two display sections 22, the measurement jig 20 may have only one display section 22. In this case, the shape of the front surface 11a of the thyroid cartilage 11 may be measured by the measurement jig 20 separately on the left and right across the imaginary line Y, illustrated in Fig. 1, on which a cut surface is to be formed in the thyroid cartilage 11.

The movable members 21 may have, although not shown, a locking convex portion or the like formed to prevent falling from the insertion hole 24 of the maintenance section 23.

As illustrated in Fig. 13, the maintenance section 23 may be divided into two. One of the divided pieces 36 to be divided into two preferably has a divided surface 36a formed with a fitting convex portion 37 and the other divided piece 36 preferably has a divided surface 36a formed with a fitting convex portion 38 corresponding to the fitting convex portion 37. Fitting of the fitting convex portion 37 and the fitting convex portion 38 allows formation of the integrated maintenance section 23 not to easily divide the divided pieces 36 while sandwiching the plurality of movable members 21.

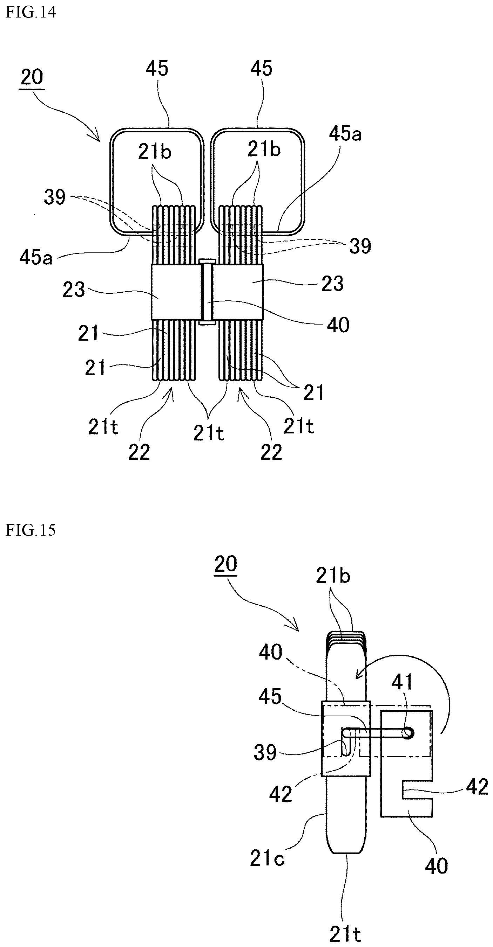

As illustrated in Fig. 14, the movable members 21 may have long holes 39, formed on the base end surface 21b side, in communication with all the movable members 21 configuring one display section 22 and a ring member 45 may be inserted into the long holes 39 to collectively bundle the plurality of movable members 21. In this case, the long holes 39 are preferably opened at the approximately same position in approximately each movable member 21 when the distal end surfaces 21t of the movable members 21 are arranged in a line and preferably opened in a length allowing sufficient relative movement of the movable members 21 in the longitudinal direction while the movable members 21 are arranged, for example, on the front surface 11a of the thyroid cartilage 11 illustrated in Fig. 1 to make a mold.

The ring member 45 is a member annularly formed with a metal or a hard resin with degradation resistance. The ring member 45 preferably has a linear area 45a to facilitate alignment of the distal end surfaces 21t of the movable members 21 while the movable members 21 are pulled out and lifted for each display section 22. The ring member 45 may allow the movable members 21 to be scattered by opening the ring.

Such a configuration allows free relative movement of each movable member 21 in the range of the long hole 39 for tracing the shape of the thyroid cartilage 11. The configuration also exhibits an effect of allowing, after tracing the shape of the thyroid cartilage 11, holding of the ring member 45 to be pulled out of the insertion hole 24 of the maintenance section 23 for each display section 22 and allowing efficient washing and the like of the display section 22 collectively without scattering each movable member 21. The configuration also exhibits an effect of, after washing the display section 22, facilitating alignment of the plurality of movable members 21 by the linear portion 45a to be inserted again into the insertion hole 24 of the maintenance section 23 and thus allowing very convenient handling.

The maintenance section 23 and the spacer 40 may be formed in a configuration, not limited to the configuration of attaching by the magnetic force of the magnets, of forming corresponding uneven shapes in the surfaces in contact with each other to tightly fit one of the maintenance section 23 and the spacer 40 to the other. Such a configuration also allows the measurement jig 20 to be used by readily combining the maintenance section 23 with the spacer 40.

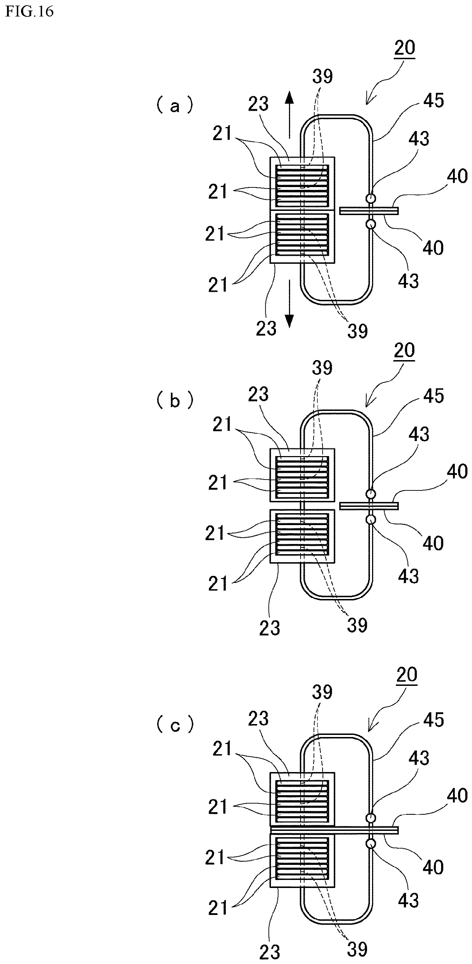

As illustrated in Fig. 15 or Figs. 16A-16C, the measurement jig 20 may have long holes 39 formed in communication with the movable members 21 and the maintenance section 23 to pass the ring member 45 and further pass the spacer 40 through the ring member 45.

In this case, the movable members 21 and the maintenance section 23 have a configuration, in addition to substantially the same configuration as any of the embodiment and the modifications described above, provided with long holes 39 to insert the ring member 45 in the thickness direction of the movable members 21 and the maintenance section 23. The long holes 39 are preferably opened in a length allowing sufficient relative movement of the movable members 21 in the longitudinal direction of the movable members 21 while the movable members 21 are arranged on the front surface 11a of the thyroid cartilage 11 illustrated in Fig. 1 to make a mold.

The spacer 40 in the present modification is formed in a plate shape with a thickness of 0.5 mm or 1 mm, facilitating adjustment of the space between the maintenance sections 23, 23.

In the thickness direction of the spacer 40, a hole 41 to insert the ring member 45 is formed. The spacer 40 has a notch formed therein to catch the ring member 45 at a position facing the position of the ring member 45 being passed through the hole 41.

It should be noted that, in the example of Figs. 16A-16C, the ring member 45 is provided with, although not necessary, stoppers 43, 43 on both sides of the spacer 40 for positioning to some extent of the spacer 40 on the ring member 45.

As illustrated in Fig. 16A, this configuration allows the measurement jig 20 in the present application example to place the spacer 40 at the position of the ring member 45 equivalent to an extension surface of the contact surface of the adjacent maintenance sections 23, 23 to interpose the spacer 40 between the maintenance sections 23, 23. Specifically, as illustrated in Fig. 16A, the two maintenance sections 23, 23 provided with the movable members 21 are aligned to make a mold of the front surface 11a at the placement site in the thyroid cartilage 11 illustrated in Fig. 1. Then, as illustrated in Fig. 16B, the maintenance sections 23, 23 are separated in accordance with the determined distance between the cut end surfaces 12a, 12a in the thyroid cartilage 11 to interpose the spacer 40 as illustrated in Fig. 15 and Fig. 16C. At this point, the spacer 40 is maintained between the maintenance sections 23, 23 by catching the ring member 45 with the notch 42 between the maintenance sections 23, 23.

The measurement jig 20 illustrated in Fig. 15 and Figs. 16A-16C thus exhibits an effect, in addition to the functions and effects described in the above embodiment, of easier handling of the measurement jig 20 composed of a plurality of parts.

It should be noted that, although Figs. 16A-16C illustrate the examples of inserting two ring members 45, the number of spacer 40 may be one or three or more. The thickness of the spacer 40 is not limited to 0.5 mm and may be formed in an appropriate dimension to facilitate adjustment of the space between the maintenance sections 23, 23.

The ring member 45 may be formed in a rectangular parallelepiped, an ellipse, or the like at least partially along the long holes 39 to direct the axis of the insertion hole 24 of the maintenance section 23 in a direction constantly orthogonal to the surface surrounded by the ring member 45. The ring member 45 partially thus configured allows prevention of unintentional rotation of the maintenance section 23 and the movable members 21 with respect to the ring member 45.

### Reference Signs List

- 11: Thyroid Cartilage
- 11a: Front Surface
- 20: Measurement Jig (Jig for Measuring Shape of Thyroid Cartilage)
- 21: Movable Member
- 21b: Base End Surface
- 21c: Side Surface
- 21t: Distal End Surface
- 22: Display Section
- 23: Maintenance Section
- 24: Insertion Hole
- 35: Groove Portion
- 39: Long Hole
- 40: Spacer
- 41: Hole
- 42: Notch
- 45: Ring Member
- M: Scale

## Claims

1. A jig for measuring a shape of thyroid cartilage comprising:
a display section having a plurality of relatively slidable movable members and configured to display a shape of thyroid cartilage by contacting distal end surfaces of the plurality of movable members with a front surface of the thyroid cartilage; and
a maintenance section configured to align and maintain the movable members in one direction and capable of fixing the slid movable members.

2. The jig for measuring a shape of thyroid cartilage according to Claim 1, wherein two of the display section are provided in the one direction with a space.

3. The jig for measuring a shape of thyroid cartilage according to Claim 2, further comprising a spacer configured to adjust a space between the display sections.

4. The jig for measuring a shape of thyroid cartilage according to any one of Claims 1 through 3, wherein the movable members are detachably provided to the maintenance section.

5. The jig for measuring a shape of thyroid cartilage according to any one of Claims 1 through 4, wherein the movable members aligned in the maintenance section have a thickness dimension of 0.5 mm or more and 5 mm or less.

6. The jig for measuring a shape of thyroid cartilage according to any one of Claims 1 through 5, wherein the maintenance section has, formed therein, an insertion hole formed with a groove portion to allow the movable members to slide or insertion holes to insert the movable members one by one.

7. The jig for measuring a shape of thyroid cartilage according to any one of Claims 1 through 6, wherein each movable member has, formed therein, a long hole extending in a direction of relative movement of the movable members to each other, and
a ring member is inserted into the long holes of the plurality of movable members configuring the display section.

8. The jig for measuring a shape of thyroid cartilage according to Claim 7 citing Claim 3, wherein
the spacer has a hole formed therein,
the spacer is rotatably maintained by the ring member while the ring member is inserted into the hole of the spacer, and
the spacer has a notch formed therein capable of engaging with an area different from an area of the ring member being inserted into the hole.

9. The jig for measuring a shape of thyroid cartilage according to any one of Claims 1 through 8, wherein a side surface or a base end surface of any of the plurality of movable members has a color different from the other movable members.

10. The jig for measuring a shape of thyroid cartilage according to any one of Claims 1 through 9, wherein each of the movable members has a side surface with a scale marked with fixed intervals and linearly extending in a horizontal direction while base end surfaces or the distal end surfaces of the movable members.
